Europäisches Patentamt

European Patent Office

Office européen des brevets

(19)

(11) Publication number: **0 203 586**
**A2**

# EUROPEAN PATENT APPLICATION

(12)

(21) Application number: **86107242.9**

(22) Date of filing: **28.05.86**

(51) Int. Cl.⁴: **A 61 K 35/74**

(30) Priority: **29.05.85 US 738978**
**14.05.86 US 863055**

(43) Date of publication of application: **03.12.86**
**Bulletin 86/49**

(84) Designated Contracting States: **AT BE CH DE FR GB IT LI LU NL SE**

(71) Applicant: **Pioneer Hi-Bred International, P.O. Box 258 6800 Pioneer Parkway, Johnston Iowa 50131 (US)**

(72) Inventor: **Hill, John E., 9501 Aurora, Des Moines Iowa 50322 (US)**
Inventor: **Marshall, William E., 701 Foster Drive, Des Moines Iowa 50312 (US)**

(74) Representative: **VOSSIUS & PARTNER, Siebertstrasse 4 P.O. Box 86 07 67, D-8000 München 86 (DE)**

(54) A composition for treating gastrointestinal disease in animals.

(57) Described is a composition for treating gastrointestinal disease in an animal containing an effective amount of <u>Lactobacillus fermentum</u> ATCC 53113 or mutants thereof.

EP 0 203 586 A2

# VOSSIUS & PARTNER

PATENTANWÄLTE

EUROPEAN PATENT ATTORNEYS

Dr. VOLKER VOSSIUS, Dipl.-Chem.
DOROTHEA VOSSIUS, Dipl.-Chem.
Dr. PAUL TAUCHNER, Dipl.-Chem.
Dr. DIETER HEUNEMANN, Dipl.-Phys.
Dr. PETER RAUH, Dipl.-Chem.
Dr. GERHARD HERMANN, Dipl.-Phys.

SIEBERTSTRASSE 4
P.O. BOX 86 07 67
8000 MÜNCHEN 86
PHONE: (089) 47 40 75
CABLE: BENZOLPATENT MÜNCHEN
TELEX: 529 453 VOPAT D
TELEFAX: (089) 47 20 01 (GR. II + III)

**0 203 586**

Our Ref.: U 617 EP
Case: 757/1:C1:EP
Pioneer Hi-Bred International

## A COMPOSITION FOR TREATING GASTROINTESTINAL DISEASE IN ANIMALS

The present invention relates to a composition for treating gastrointestinal disease in animals such as pigs, cows, sheep, goats or horses.

Gastrointestinal disease is a major cause of economic loss in domestic animals. Often the effect of such illness is greatest at the neonatal stage of development when the animals' own immune system is not fully capable of adequately protecting them from pathogens which may be present in their environment. Many times gastrointestinal diseases lead to death or a slowed growth rate, resulting in a longer time to market, and increased expense to the farmer. Even if an animal recovers from gastrointestinal disease the days of growth that are lost and the associated delay when the animal is ready for market can be costly for the farmer. It is estimated that for each day of growth that is lost the animal will require two or three more days to reach market weight. Although gastrointestinal diseases may be of viral or bacterial etiology, those caused by <u>Escherichia coli</u> are among the most common and debilitating.

Those enterotoxigenic strains of <u>E. coli</u> which cause gastrointestinal disease are apparently capable of binding to the intestinal epithelial cells of a susceptible animal through bacterial adhesins coded for by a plasmid. The pathogenic effect of these <u>E. coli</u> is then caused by the production of an enterotoxin which is also coded for by a plasmid carried by the organism.

Various attempts have been made to prevent or eliminate gastrointestinal disease in animals. For example, Farr, United States Patent 3,953,609, discloses a method of orally feeding animals <u>Lactobacillus lactis</u> NRRL B-5628 to restrict the growth of other digestive system bacteria. The patentee states that this organism is particularly useful in the treatment of scouring, diarrhea or colibacillosis in baby pigs and other newborn animals by reducing the intestinal <u>E. coli</u> population. The patentee also states that this strain can be used in farrowing sows to reduce their intestinal bacteria, which in turn will reduce such bacterial populations in newborn pigs.

Henry <u>et al.</u>, United States Patent 3,320,130, disclose a process for the preparation of a medicament containing a mixture of Lactobacillus acidophilus and nonpathogenic E. coli. Henry et al. state that the medicament of the invention is useful in treatment of colitis, gastroenteritis and enterocolitis in infants and adults.

In Hata et al., United States Patent 4,314,995, a process for treating infectious disease which involves administering Lactobacillus FRI strain No. 1946, 2779, 2780, 2781 or 2782 with or without antibiotics is disclosed. It is stated that this process can be used for treating patients with gastritis and enteritis, but it is recommended that the treatment be used in combination with antibiotics for these diseases.

Nouvel, United States Patent 3,369,969, discloses a process of preparing a medicament composed of nonpathogenic colibacilli resistant to antibiotics with or without the presence of bifidus or clostridium bacteria. It is stated that this medicament can be used for the treatment of illnesses connected with a lack of balance of the intestinal flora. The patentee further discloses the use of this medicament in treating patients with diarrhea.

In a study reported by Barrow et al. (Journal of Applied Bacteriology, 48: 147 (1980)), the authors examined the attachment of strains of Lactobacillus fermentum and Streptococcus salivarius to pig gastric epithelium. The authors report that a statistically significant reduction in E. coli count was produced in both the stomach and the duodenum by feeding a combination of Lactobacillus fermentum Strain 14 and Streptococcus salivarius Strain 312 for four days. However, when L. fermentum strain 14 alone was administered for four days, there was a significant reduction in the numbers of E. coli only in the stomach of these animals. The authors further state that this treatment is unlikely to be effective in combating diarrhea caused by E. coli since the count of E. coli in the duodenum was still greater than $10^6$ per gram of tissue. The authors speculate that if the antibacterial effect of strain 14 could be increased, as by feeding the animals larger numbers of strain 14 or by administration of a concentrated form of the inhibitory factors produced by strain 14, some effect on scouring due to E. coli should follow.

Muralidhara et al. (Journal of Food Protection, 40: 288, 1977) describe the effects of feeding Lactobacilli on the flora of intestinal tissue and feces from piglets. These authors found that administration of a strain of Lactobacillus lactis concentrate alone prior to challenge with an enterotoxigenic strain of E. coli did not protect animals from scours. Complete protection from scours was found only in those animals which received sow colostrum in additon to Lactobacillus lactis prior to infectious challenge.

In sum, the background art indicates that although previous investigators had attempted the use of bacterial prophylaxis to prevent or eliminate gastrointestinal disease, the organisms used in this manner were only partially effective. Thus, there is a continuing need for a simple, nontoxic, non-antibiotic treatment of gastrointestinal disease in animals.

In the invention, an animal having, or at risk of having, gastrointestinal disease is given a therapeutic amount of a composition containing <u>Lactobacillus</u> <u>fermentum</u> ATCC 53113. It is speculated that this organism appears capable of binding to specific receptors on the surface of gut-associated epithelial cells of the animal being treated, thereby suppressing the pathogenic effect of enterotoxigenic organisms binding to the same receptors. It is also possible that this organism produces substances which inhibit the growth or attachment of these enterotoxigenic pathogens.

Alternatively, it is also thought that <u>L. fermen-</u> <u>tum</u> ATCC 53113 may protect an animal from enterotoxigenic organisms by stimulating the immune system of the animal or by helping to maintain a healthy gut such that the animal is less likely to develop gastrointestinal disease.

Thus, the present invention provides a composition for treating gastrointestinal disease in an animal which comprises an effective amount of <u>Lactobacillus</u> <u>fermentum</u> ATCC 53113 or mutants thereof.

The invention also provides the strain/ and mutants thereof. <u>Lactobacillus</u> <u>fermentum</u> ATCC 53113 was deposited with the American Type Culture Collection under the Budapest Treaty.

BRIEF DESCRIPTION OF THE PREFERRED EMBODIMENTS

Gastrointestinal diseases are those relating to or affecting the stomach and the intestines.

According to the invention, an animal which has, or is in danger of having, a gastrointestinal disease is given a therapeutically effective amount of <u>Lactobacillus</u> <u>fermentum</u> ATCC 53113 or an effective mutant thereof. In a preferred embodiment of the invention a therapeutically effective amount of <u>L. fer-</u> <u>mentum</u> ATCC 53113 is administered to weanling animals.

A23.10                                                    5/8/86

The composition of the present invention is highly desirable in that the organism of the invention is nonpathogenic and should thereby render unlikely the occurrence of any deleterious effects due to this organism. Also of significance is the fact that the composition for treating gastrointestinal disease according to the invention does not necessitate the supplemental use of antibiotics and relies rather upon "natural" mechanisms of controlling the infectious organism, in preventing the infectious organism from binding to gut associated tissue. This aspect of the invention is significant in the face of growing public concern over the presence of antibiotics in meat, and the effect that these antibiotics may have on the health of the general population.

The organism of the invention was isolated from the gut of a healthy newborn pig. The organism effective in suppressing gastrointestinal disease is Lactobacillus fermentum ATCC 53113 or effective mutants thereof. Such mutants are considered equivalents to the parent strain.

It is well known to those of ordinary skill in the art that spontaneous mutation is a common occurrence in microorganisms and that mutations can be intentionally produced by a variety of known procedures. For example, mutants can be induced using chemical, radioactive, and recombinant techniques. As shown in Table I chemical mutagens can be divided into four main groups based upon their activity.

## Table I

| ACTIVITY | EXAMPLES |
|---|---|
| Base Analogs | 5-bromouracil, 2-aminopurine |
| Deaminating Agents | nitrous acid, hydroxylamine |
| Alkylating Agents | ethyl ethanesulfonate, nitrosoguanidine |
| Acridine Derivatives | acridine orange, ethidium bromide |

Any of these can be used in the present invention.
Radiation induced mutations can be caused by such agents as ultraviolet light, and x-rays. The primary mechanism by which mutations may be caused result from excision or post replication repair by recombination.

Additionally, mutations can also be produced by recombinant techniques using restriction endonucleases. Use of this technique is especially valuable to allow the deletion or insertion of large DNA fragments.

Regardless of the manner in which the mutations are induced the critical issue is that the mutants function therapeutically as described for the parent strain. In other words, the present invention includes mutations resulting in such minor changes as, for example, minor taxonomic alterations such as the fermentation of certain sugars. In addition, the present invention includes mutations wherein the transfer of genetic material enables the mutant to phenotypically express multiple determinants capable of binding to different gut epithelial cell receptors where various gastrointestinal pathogens attach.

The gastrointestinal diseases for which the present invention is effective can be any in which the underlying etiology is microbial, for example, bacterial or viral in nature. Alternatively, the present invention is also useful in animals where the normal gut flora has been eliminated or unbalanced as, for example, following severe viral gastroenteritis or high dose antibiotic therapy in order to aid in the restoration of the normal gut flora and prevent colonization by opportunistic pathogens.

Among the gastrointestinal diseases which can be treated using the present invention are diarrhea, colibacillosis, and scours. Diarrhea encompasses many different etiologic agents, among those being bacterial and viral in nature. Such disease is characterized by the abnormal frequency and the liquidity of the fecal discharges. Colibacillosis, as used herein, denotes gastrointestinal disease due to infection with Escherichia coli. Scours is a severe form of gastrointestinal disease. This disease is a type of diarrhea commonly found in such domestic animals as pigs, cows, sheep, goats, and horses. Often during the course of this disease, the fecal discharge is accompanied by blood in the later stages and is marked by fever, dehydration, and depression. Commonly found in newborn animals, scours is often fatal and is a major source of economic loss to farmers. Newborn animals are especially susceptible to scours-causing pathogens because these animals have not had sufficient time to develop an immune response to the pathogen. Instead, these neonatal animals are dependent upon maternal colostrum for passive immune protection to disease.

Unfortunately, the degree of protection offered by the colostrum varies considerably from animal-to-animal and may depend, in part, on the age and immunologic experience of the mother. Additionally, the fact that neonatal animals have not yet established a normal gut flora also makes them particularly susceptible to opportunistic pathogenic infection.

Young animals are also particularly susceptible to gastrointestinal disease after they have been weaned from the mother and are adjusting to a solid-feed diet. In this situation the animal has been removed from the comfort and protection of its mother and put in the company of strange animals. The digestive tract has been forced to go from highly digestible milk to a less digestible vegetable substitute (for example, corn and soy beans). In addition, the weaned animal has to learn how to eat the new feed. When an animal is weaned, the two major concerns of the farmer are getting the animal to eat the new feed and to stay healthy. Later, when the animal has adjusted to the new food ration, the farmer worries about weight gain and feed efficiency. Stress and dietary changes have been shown to cause alteration in the microflora of the digestive tract. Because the protective microflora of the gut has been disrupted, these animals are more likely to develop scours. The scouring may be due to bacteria, viruses, or both.

During the stage of the animal's life following weaning, scours is most likely due to viral infections. It is also possible that enhanced susceptibility to gastrointestinal disease at this time in the animal's life is caused by the loss of protective maternal antibody passively acquired in milk during nursing which is eliminated before the animal has developed its own active protective immunity to the various gastrointestinal etiologic agents. It is conceivable that in a state of microfloral or immunological transition the gut is more susceptible to opportunistic pathogenic infection.

However, regardless of the mechanism by which the animal develops a particular gastrointestinal disease it can be ameliorated using L. fermentum ATCC 53113.

Protection from gastrointestinal disease can most easily be accomplished by feeding L. fermentum ATCC 53113 or compositions containing this organism to the animal in which protection is sought.

Compositions may be in a liquid, lyophilized, or gel form and may contain additional bacterial strains for the treatment of gastrointestinal disease. In solid dosage forms, the composition may comprise the organism of the invention together with a pharmaceutical carrier. The pharmaceutical carrier may be in the nature of an aqueous or nonaqueous liquid or a solid. In solid dosage forms, the composition may contain such inert diluents as sucrose, lactose, starch, or vermiculite as well as a lubricating agent. These lubricating agents aid in the passage of the compositions through the gut. In the case of capsules, tablets and pills, the unit dosage forms may also comprise buffering agents. Other forms of oral administration may also be prepared with an enteric coating which would prevent dissolution of the composition until reaching the intestines.

Compositions according to this invention would contain $10^3$-$10^{12}$ viable organisms/gm, more preferably $10^4$-$10^{10}$ viable organisms/gm, most preferably $10^5$-$10^8$ viable organisms/gm.

Liquid dosage forms for oral administration will generally comprise an enterically coated capsule containing the liquid dosage form. Suitable forms include emulsions, suspensions, solutions, syrups, and elixirs containing inert diluents commonly used in the art, such as purified water, sugars, polysaccharides, silicate gels, gelatin, or an alcohol. These inert diluents do not actively participate in the therapeutic effect of the invention. Besides the inert diluents, such compositions can also include adjuvants, such as wetting agents, emulsifying and suspending agents, and sweetening, flavoring, and perfuming agents.

The dose ranges for a given animal would vary depending on the weight and concomitant administration of antibiotics. The dose could be administered as either single or multiple dosages and would contain $10^3$-$10^{12}$ viable organisms/dose, more preferably $10^4$-$10^{10}$ viable organisms/dose, most preferably $10^5$-$10^8$ viable organisms/dose.

Protection from gastrointestinal disease can also be accomplished by immunizing an animal with a vaccine composed of whole cells and/or cell extracts of L. fermentum ATCC 53113. These cell extracts can be prepared by various techniques known to those of skill in the art. The important aspect of these extracts being that they protectively stimulate the immune system of the animal to ameliorate the effect of any subsequent exposure to gastrointestinal disease etiologic agents. This vaccine can be administered either enterally or parenterally in immunologically effective amounts. The term "immunologically effective amount," as used in the invention, is meant to denote that amount of vaccine which is necessary to sensitize the immune system of the animal to ameliorate the effect of gastrointestinal disease.

Vaccination by enteral administration can be accomplished as described supra for the oral presentation of viable organisms.

Parenteral vaccination of an animal can be done by immunizing an animal by subcutaneous, intramuscular, or intravenous injection, or by nasopharyngeal, mucosal, or transdermal absorption. Preparations for parenteral administration include sterile or aqeuous or non-aqueous solutions, suspensions, and emulsions. Examples of non-aqeuous solvents are propylene glycol, polyethylene glycol, vegetable oils such as olive oil, and injectable organic esters such as ethyl oleate. Carriers for occlusive dressings can be used to increase skin permeability and enhance vaccine absorption. Besides the inert diluents, such compositions can also include adjuvants, wetting agents, emulsifying and suspending agents.

It is also possible for the vaccine preparations to include an adjuvant. Adjuvants are substances that can be used to non-specifically augment a specific immune response. Normally, the adjuvant and the antigen are mixed prior to presentation to the immune system, or presented separately, but into the same site of the animal being immunized. Adjuvants are loosely divided into several groups based on their composition. These groups include oil adjuvants (for example Freund's Complete and Incomplete), mineral salts (for example, $AlK(SO_4)_2$, $AlNa(SO_4)_2$, $AlNH_4(SO_4)$, silica, kaolin, and carbon), polynucleotides (for example, poly IC and poly AU acids), and certain natural substances (for example, wax D from <u>Mycobacterium</u> <u>tuberculosis</u>, as well as substances found in <u>Corynebacterium</u> <u>parvum</u>, <u>Bordetella</u> <u>pertussis</u>, and members of the genus <u>Brucella</u>). Among those substances particularly useful as adjuvants are the saponins such as, for example, Quil A (Superfos A/S, Denmark).

Many different techniques exist for the timing of the immunizations with the vaccine of the invention when a multiple immunization is utilized. It is possible to use the vaccine of the invention more than once to increase the level and diversity of expression of the immune response of the animal which is expressed. Typically, if multiple immunizations are given, they will be spaced 1-2 months apart.

The dose ranges for a given animal would vary depending on the weight and concomitant administration of antibiotics. The dose could be administered as either single or multiple dosages and would contain $10^3$-$10^{12}$ organisms/dose, more preferably $10^4$-$10^{10}$ organisms/dose, most preferably $10^5$-$10^8$ organisms/dose.

Alternatively, an animal can be administered the equivalent of these concentrations of organisms where the values are expressed by some other measurement such as, for example, total protein concentration or in cell fragment concentration.

The compositions of the invention can include, in addition to <u>Lactobacillus fermentum</u> ATCC 53133, mutants of this organism or such other common gut organisms as, for example, bifidus or clostridial bacteria.

The invention also relates to a method for preparing a medicament or pharmaceutical composition comprising the components of the invention, the medicament being used for the treatment or prevention of gastrointestinal disease.

Those of ordinary skill in the art will know of other suitable diluents and dosage forms, or will be able to ascertain such, using routine experimentation. Further, the administration of the various compositions can be carried out using standard techniques common to those of ordinary skill in the art.

The above disclosure generally describes the present invention. A more complete understanding can be obtained by reference to the following specific example which is provided herein for purposes of illustration only and is not intended to be limiting unless otherwise specified.

## EXAMPLE 1

<u>Lactobacillus fermentum</u> (ATCC 53113) is a Gram positive rod which was isolated from the gut of a healthy neonatal pig. The fermentation profile of this organism is shown in Table II. This profile was performed using a commercially available test system (CHL kit, DMS Laboratories, Darts Mill, Flemington, New Jersey).

A23.10                                                    5/8/86

### Table II

| Substrate | Reaction | Substrate | Reaction |
|---|---|---|---|
| glycerol | − | salicin | − |
| erythritol | − | cellobiose | − |
| D-arabinose | − | maltose | + |
| L-arabinose | − | lactose | + |
| ribose | + | melibiose | + |
| D-xylose | − | saccharose | + |
| L-xylose | − | trehalose | − |
| adonitol | − | inuline | − |
| beta-methyl-xyloside | − | melezitose | − |
| galactose | + | D-raffinose | + |
| D-glucose | + | starch | − |
| D-fructose | − | glycogen | − |
| D-mannose | − | xylitol | − |
| L-sorbose | − | beta-gentiobiose | − |
| rhamnose | − | D-turanose | − |
| dulcitol | − | D-lyxose | − |
| inositol | − | D-tagatose | − |
| mannitol | − | D-fucose | − |
| sorbitol | − | L-fucose | − |
| alpha-methyl-D-mannoside | − | D-arabitol | − |
| alpha-methyl-D-glycoside | − | L-arabitol | − |
| N-acetyl-glucosamine | − | gluconate | + |
| amygdalin | − | 2-keto-gluconate | − |
| arbutin | − | 5-keto-gluconate | − |
| esculin | − | | |

Strain ATCC 53113 was grown in MRS broth supplemented with DL-threonine (Chassy et al., Journal of Bacteriology, 127: 1576-1578, 1976) and the plasmid profile determined as described by Anderson et al., Applied and Environmental Microbiology, 46: 549-552 (1983). The size of the plasmids was established using horizontal electrophoresis in agarose (0.6%) with a TRIS-borate buffer (Meyers et al., Journal of Bacteriology, 127: 1529-1537, 1976). ATCC 53113 was found to have six plasmids of various sizes as shown in Table III.

## Table III
### Size[a] of Plasmids Present in Lactobacillus fermentum, ATCC 53113

27.7

8.7

4.3

3.5

2.6

2.1

[a]  in Megadaltons

## EXAMPLE 2

The effects of the methods and compositions of the invention were investigated in newborn pigs. Five newborn pigs were removed from their mother immediately following birth. These animals were not permitted to nurse from the mother so that no passive immunity was obtained via maternal colostrum. The animals were divided into groups and housed separately. Animals in Group I orally received $10^{10}$-$10^{11}$ Lactobacillus fermentum ATCC 53113 in sterile skim milk. Eight hours after receipt of this therapeutic dose, these animals were challenged using $10^8$ E. coli K-99 (NADC 1472) in sterile skim milk. This organism is an enteropathogen known to cause scours in pigs. Group II animals were not pretreated with Lactobacillus fermentum ATCC 53113, but were orally infected with $10^8$ E. coli K-99 (NADC 1472). The animal in Group III served as a control and was not orally inoculated with any organisms. All animals were monitored as to their time of death due to scours. The results obtained are shown in Table IV.

## Table IV

| Group | N | ATCC 53113 Therapeutic Dose | K-99 Infectious Challenge Dose | Time to Death By Scours (Hrs) |
|---|---|---|---|---|
| 1 | 2 | $10^{10}$-$10^{11}$ | $10^8$ | No death |
| 2 | 2 | --- | $10^8$ | 32 |
| 3 | 1 | --- | --- | No death |

The animals in the experiment which were pretreated with the organism of the invention did not die from scours when challenged using a known enteropathogenic strain of E. coli. This is in sharp contrast to the animals in group II which quickly developed the characteristic symptoms of scours and died within 32 hours of exposure to E. coli K-99 (NADC 1472). Survival of the control animal shows that the death of the animals in group II was due to infectious challenge and not due to other environmental factors which may have been present during the experiment.

## EXAMPLE 3

Three-week old pigs (mixed breeds and sex) were removed from their mothers while still nursing and prior to exposure to solid food. The animals were randomly assigned to either the control or experimental group, ear tagged, and orally inoculated with 2-ml of a placebo gel (control group) or a gel containing L. fermentum ATCC 53113 ($6x10^5$ organisms) in the gel. All animals were housed individually in wire cages elevated above the floor. Feed and water were given ad libidum. Weights and diarrhea observations were recorded daily for a period of 21 days and feed consumption measured weekly. The numbers of animals which had scours on each day of the experiment are shown in Table V.

## TABLE V

Effect of Administration of L. fermentum ATCC 53113 on the
Kinetics of Endemic Scours Infection

NUMBER OF
ANIMALS SCOURING AT EACH DAY

| GROUP | 1 | 2 | 3 | 4 | 5 | 6 | 7 | 8 | 9 | 10 | 11 | 12 | 13 | 14 | 15 | 16 | 17 | 18 | 19 | 20 | 21 |
|-------|---|---|---|---|---|---|---|---|---|----|----|----|----|----|----|----|----|----|----|----|----|
| Control | 0 | 0 | 0 | 0 | 0 | 0 | 1 | 1 | 1 | 1 | 1 | 2 | 2 | 2 | 4 | 4 | 4 | 3 | 4 | 3 | 4 |
| Experimental | 0 | 0 | 0 | 2 | 0 | 0 | 0 | 1 | 1 | 1 | 1 | 1 | 1 | 1 | 0 | 0 | 0 | 0 | 0 | 0 | 0 |

In general, animals receiving L. fermentum ATCC 53113 showed a much lower incidence, and severity, of scouring. Of the nine animals in the control group that developed scours four had symptoms lasting 7 days or longer.

The results of this experiment are summarized in Table VI.

### TABLE VI

Effect of L. fermentum ATCC 53113 on Scours

| Groups | N | Average lbs. Gained/pig | Average lbs. Feed Consumed | Total Days Scouring | Cases of Scours | Average[b] Duration of Scours |
|--------|---|------------------------|---------------------------|---------------------|-----------------|------------------------------|
| Control | 15 | 7.25(.73)[a] | 14.77(1.82) | 37 | 9 | 5 |
| Experimental | 15 | 7.01(.69) | 15.24(1.21) | 9 | 4 | 2 |

[a] STD error of mean

[b] Days

The four animals which still had scours on day 21 were sacrificed and a diagnosis of rotavirus as the etiologic agent was confirmed by enzyme-linked immunosorbent assay (ELISA).

The animals in the experimental group which were treated with gel containing ATCC 53113 cells were more resistant to endemic infection due to rotavirus than were animals in the control group which did not receive ATCC 53113 (4 animals vs 9 animals). Further, animals in the experimental group that did develop rotavirus infection, scoured for a much shorter period of time than those animals developing rotavirus infection in the control group (2 day vs 5 days). Finally, the total pig-days of scouring was much greater for the control group than for the experimental groups (37 days vs 9 days).

## Example 4

Another experiment was done in the same format as described _supra_ in Example 3 except that in this experiment the animals received a second dose of placebo gel (control group) or _L. fermentum_ ATCC 53113 containing gel (experimental group) on the evening of day 14 of the experiment. The numbers of animals which had scours on each day of the experiment are shown in Table VII.

## TABLE VII

Effect of Multiple Administration of L. fermentum ATCC 53113 on the
Kinetics of Endemic Scours Infection

NUMBER OF
ANIMALS SCOURING AT EACH DAY

| GROUP | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| Control | 0 | 0 | 1 | 1 | 3 | 4 | 2 | 0 | 0 | 0 | 0 | 0 | 1 | 4 | 6 | 6 | 0 | 0 | 0 | 0 | 0 |
| Experimental | 0 | 0 | 0 | 0 | 0 | 0 | 0 | 0 | 0 | 0 | 1 | 0 | 0 | 4 | 0 | 0 | 0 | 0 | 0 | 0 | 0 |

0 203 586

The incidence of scours in the control animals occurred at two time periods. Five cases of scours occurred in the control group early in the experiment during days 3-7. A second outbreak of seven cases of scours occurred on days 3-16. Most of the cases of scours that were encountered by animals in the experimental group were seen on day 14 of the experiment. On the evening of day 14 the animals in both groups received a second dose of placebo gel (control group) or gel with L. fermentum (experimental group). At this time four animals in each group had active scours infections. Those animals receiving the gel containing L. fermentum showed no symptom of scours on day 15. In contrast, in the control group three of the four animals which had scours on day 14 were still infected on day 15 and, in addition, three more animals in the control group had developed scours infection. The overall results of this experiment are summarized in Table VIII.

## TABLE VIII

### Effect Multiple Treatment with of
### L. fermentum ATCC 53113 on the Development of Scours

| Groups | N | Average lbs. Gained/pig | Average lbs. Feed Consumed | Total Days Scouring | Cases of Scours | Average[e] Duration of Scours |
|---|---|---|---|---|---|---|
| Control | 19[a] | 6.59(0.66)[b] | 14.43(1.03) | 27 | 14[d] | 2 |
| Experimental | 20 | 7.00(0.51) | 17.07(0.88)[c] | 5 | 5 | 1 |

[a] One animal died during trial

[b] STD error of mean

[c] Significantly different at 0.06 level

[d] Two animals in the control group developed scours twice

[e] Days

The animals in the experimental group did not develop the early scours infections which were seen in the control animals on days 3-7. Those cases of scours which did occur in the experimental group were later in the experiment (day 14) and coincided with a second outbreak of scours in the control group. In general, animals in the experimental group performed much better than those in the control group. The experimental animals showed fewer cases of scours (5 vs 14) and when the experimental animals did develop scours the duration of illness was only half that of the control animals which developed scours. In addition, the experimental group had far fewer total pig-days of scouring than did the control group (5 days vs 27 days). The fact that the experimental animals consumed a significantly greater amount of solid feed than did the control animals indicates a more rapid adjustment to solid food in the experimental animals (17 lbs. vs 14 lbs).

This study shows that administration of L. fermentum ATCC 53113 can not only delay or prevent the likelihood of an animal developing scours, but also, as shown here by the administration of a second dose on day 14, can accelerate the remission of scours in those animals with active scours infections.

CLAIMS:

1. A biologically pure culture Lactobacillus fermentum
   ATCC 53113 or gastrointestinal disease-preventing mutants
   thereof.

2. A composition comprising the Lactobacillus fermentum
   of claim 1 together with a pharmaceutical carrier.

3. The composition of claim 2 wherein said carrier is
   sucrose, lactose, or starch.

4. The composition of claims 2 and 3 wherein said carrier
   contains a lubricating agent.

5. The composition of claims 2 to 4 wherein said composition
   is contained in an enteric coating.

6. The composition of claims 2 to 5 wherein said composition
   contains $10^3$-$10^{12}$ viable organisms/g.

7. The composition of claim 2 wherein the composition is a
   vaccine for immunizing the animal.

8. The composition of claim 7, wherein said composition
   contains an adjuvant, e.g. a saponin derivative, such
   as Quil A.

9. The composition of claims 2 to 8, wherein the composition
   comprises additional bacterial strains.

10. Lactobacillus fermentum ATCC 53113 for use in the
    treatment of gastrointestinal disease in an animal.